# EUROPEAN PATENT APPLICATION

(11) **EP 4 389 175 A1**
(43) Date of publication of application: **26.06.2024**
(21) Application number: 22306963.4
(22) Date of filing: 20.12.2022
(51) Int. Cl.: A61M 5/28, A61M 5/32

(54) **SAFETY ASSEMBLY FOR MOUNTING ONTO A SYRINGE**

(71) Applicant: Becton Dickinson France, 38800 Le Pont-de-Claix (FR)
(72) Inventor: MILLS, Freddy, 38000 Grenoble (FR)
(74) Representative: Germain Maureau

(57) **Abstract**

The assembly comprises :a syringe barrel (12) having a needle (11), a tubular body (2) defining an enclosure, said barrel (12) extending at least partially within said enclosure; a safety shield (3) for the needle (11), longitudinally movable with respect to the tubular body (2) between a retracted position and an activated position; a spring (4) urging said safety shield (3) towards said activated position; a first locking mechanism (31) for locking the safety shield (3) in the retracted position, wherein the first locking mechanism (31) is manually releasable by a user so as to let the safety shield (3) reach the activated position, wherein the safety shield (3) is able to rotate within the body (2), and has a distal part (302) axially protruding from a distal part (202) of the body (2) for gripping the safety shield (3); the first locking mechanism (31) being configured to release the safety shield (3) when the safety shield (3) is rotated from a first angular position to a second angular position.

## Description

### FIELD OF THE INVENTION

The present invention relates to a needle shielding system, and precisely to a syringe assembly for use in medication delivery, comprising a shield for enclosing a syringe needle after drug delivery completion.

### BACKGROUND OF THE INVENTION

In this application, the distal end of a component or of a device is to be understood as meaning the end furthest from the user's hand and the proximal end is to be understood as meaning the end closest to the user's hand. Likewise, in this application, the "distal direction" is to be understood as meaning the direction of injection, with respect to the safety device or syringe of the invention, and the "proximal direction" is to be understood as meaning the opposite direction to said direction of injection, that is to say the direction towards the user's hand.

Pre-fillable or prefilled syringes, usually comprise a hollow tubular body or barrel forming a container for a medical product. This tubular body comprises a distal end in the form of a longitudinal tip defining an axial passageway through which the medical product is expelled from the container. The distal end is equipped with a needle for injection of the medical product into an injection site.

It is of great importance that the patients and users are protected from any risk of needle stick injuries, particularly between the moment that injection is finished and the discarding of the drug delivery device.

Needle shielding devices can be of the so called active type where a shielding device is activated by a user after injection. An example of shielding system is Becton Dickinson Preventis^{™} needle shielding system which enables one-handed, controlled activation that automatically shields the needle. After the injection, a further pressure on the piston rod triggers the shielding as disclosed for example by EP1397170.

### SUMMARY OF THE INVENTION

This proves to be satisfactory, but it could be desirable that the activation force was completely independent from the injection movement and could be adjusted for usability purpose for instance to allow the shielding even after a partial injection (which could be required in some cases because of the age or the weight of the patient).

According to one aspect, the invention concerns a syringe assembly for use in medication delivery comprising:
- a syringe barrel having a needle at a distal end of the barrel;
- a tubular body defining an enclosure, said barrel extending at least partially within said enclosure;
- a safety shield for the needle, longitudinally movable with respect to the tubular body between a retracted position and an activated position;
- a spring urging said safety shield towards said activated position;
- a first locking mechanism for locking the safety shield in the retracted position, wherein the first locking mechanism is manually releasable by a user so as to let the safety shield reach the activated position.
- wherein the safety shield is able to rotate within the body, and has a distal part axially protruding from a distal part of the body for gripping the safety shield;
- the first locking mechanism being configured to release the safety shield when the safety shield (3) is rotated from a first angular position to a second angular position.

In some embodiments, the syringe assembly comprises a second locking mechanism for permanently and non-reversingly locking the safety shield in the activated position after the first locking mechanism is released.

In some embodiments, the second locking mechanism is configured to receive and then hold a proximal part of the safety shield.

In some embodiments, the second locking mechanism comprises first retaining features on an outer face of said proximal part of the safety shield which are forcedly deformed when the safety shield moves from the retracted position towards the activated position.

In some embodiments, the second locking mechanism further comprises second retaining features on an inner face of the distal part of the body, which are configured to interact with said first retaining features when the safety shield moves from the retracted position towards the activated position.

In some embodiments, the distal part of the safety shield presents texture features for increasing grip.

In some embodiments, the needle is covered by the safety shield in the activated position and the needle is not covered by the safety shield in the retracted position.

In some embodiments, the first locking mechanism comprises complementary first and second mating surfaces respectively on the safety shield and on the body.

In some embodiments, the first mating surface is also part of the first retaining features.

In some embodiments, the syringe assembly comprises a prefilled syringe comprising said barrel, said needle, a stopper and a plunger rod.

According to another aspect, the invention concerns a shielding system for a syringe for use in medication delivery comprising a barrel and a needle at a distal end of the barrel, said shielding system comprising:
- a tubular body defining an enclosure, said barrel extending at least partially within said enclosure;
- a safety shield for the needle, longitudinally movable with respect to the tubular body between a retracted position and an activated position;
- a spring urging said safety shield towards said activated position;
- a first locking mechanism for locking the safety shield in the retracted position, wherein the first locking mechanism is manually releasable by a user so as to let the safety shield reach the activated position;
- wherein the safety shield is able to rotate within the body, and has a distal part axially protruding from a distal part of the body for gripping the safety shield;
- the first locking mechanism being configured to release the safety shield when the safety shield is rotated from a first angular position to a second angular position.

According to another aspect , the invention concerns a method for operating a syringe assembly or a shielding system, comprising a step of rotating the safety shield from the first angular position to the second angular position to trigger release of the first locking mechanism so that the spring moves the safety shield from the retracted position towards the activated position.

### BRIEF DESCRIPTION OF THE DRAWINGS

The invention and the advantages arising therefrom will clearly emerge from the detailed description that is given below with reference to the appended drawings as follows:
FIG. 1 is a top perspective view of an embodiment of a syringe assembly according to the present invention, with a needle safety shield in a retracted position;
FIG. 2 is a top perspective view of the syringe assembly shown in FIG. 1, with the needle safety shield in an activated position;
FIG. 3 is a view of the locking mechanisms of the syringe assembly (when in the state of FIG. 1;
FIG. 4 is a view of the locking mechanisms of the syringe assembly (when in the state of FIG. 2);

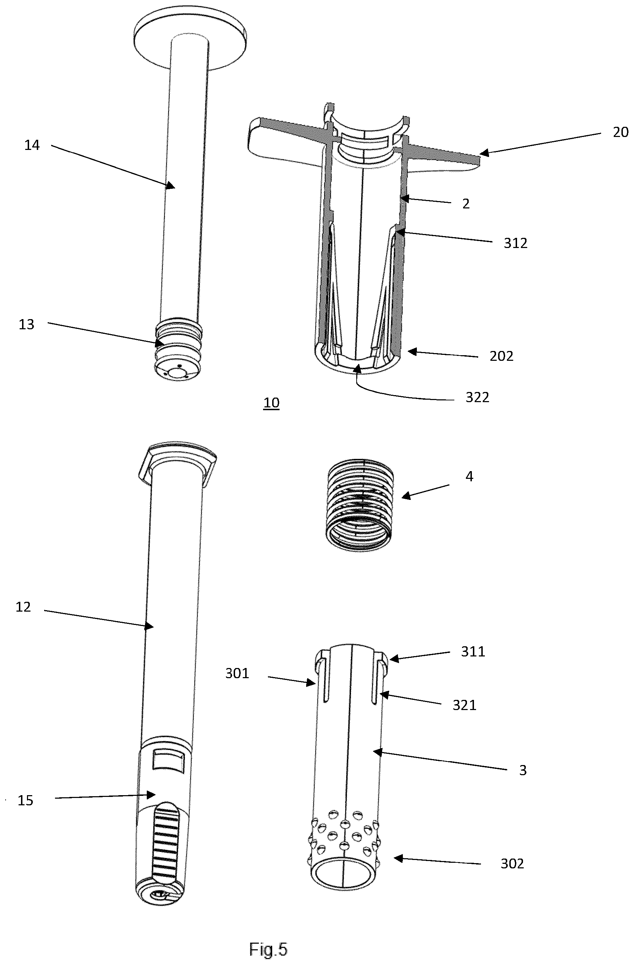

FIG. 5 is an exploded view of the syringe assembly shown in FIG. 1.

### DETAILED DESCRIPTION OF EXEMPLARY EMBODIMENTS

The different features of the embodiments can be used in combination with and used with other embodiments as long as the combined parts are not inconsistent with or interfere with the operation of the device and assembly. This invention is not limited in its application to the details of construction and the arrangement of components set forth in the following description or illustrated in the drawings. The embodiments herein are capable of being modified, practiced or carried out in various ways. The phraseology and terminology used herein is for the purpose of description and should not be regarded as limiting. The use of "including," "comprising," or "having" and variations thereof herein is to encompass the items listed thereafter and equivalents thereof as well as additional items. Unless limited otherwise, the terms "connected," "coupled," and "mounted," and variations thereof herein are used broadly and encompass direct and indirect connections, couplings, and mountings. In addition, the terms "connected" and "coupled" and variations thereof are not limited to physical or mechanical connections or couplings. Further, terms such as distal, proximal, up, down, bottom, and top are relative, and are to aid illustration, but are not limiting. The embodiments are not intended to be mutually exclusive so that the features of one embodiment can be combined with other embodiments as long as they do not contradict each other. Terms of degree, such as "substantially", "about" and "approximately" are understood by those skilled in the art to refer to reasonable ranges around and including the given value and ranges outside the given value, for example, general tolerances associated with manufacturing, assembly, and use of the embodiments. The term "substantially" when referring to a structure or characteristic includes the characteristic that is mostly or entirely present in the structure.

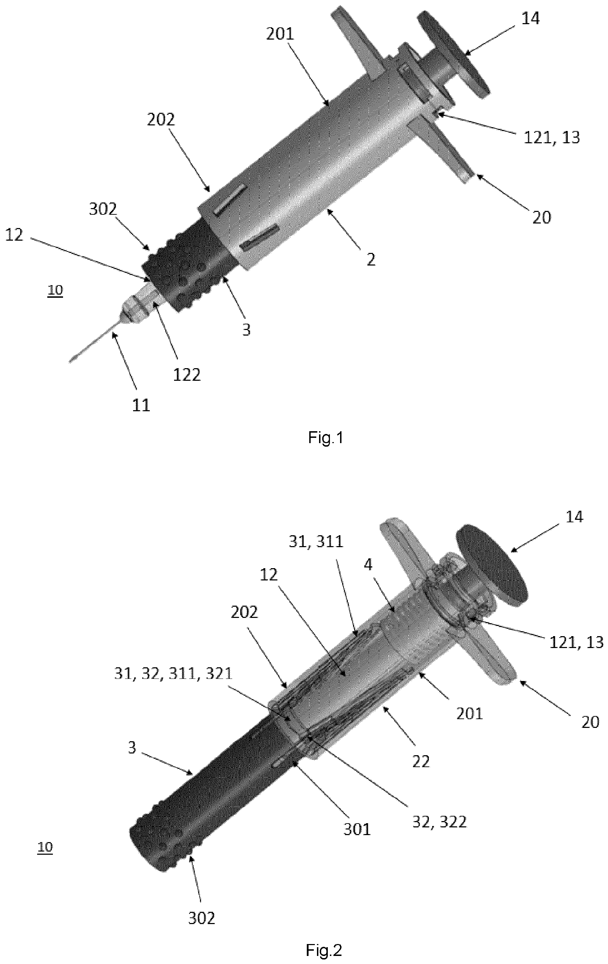

Figures 1 and 2 illustrate perspective views of a syringe assembly 10 in accordance with an embodiment of the present invention, respectively in a first state and a second state.

Said syringe assembly 10 is for use in medication delivery (in particular hypodermic injection) and preferably comprises a complete syringe 1 (in particular a pre-fillable syringe, which typically contains a single dose of medication such as such as 0.3 ml, 0.5 ml or 1.0 ml), coupled to a tubular body 2 which enables the shielding function. Syringes are typically comprised of a barrel 12, having a proximal end 121 and a distal end 122 with respect to said longitudinal axis; a needle 11 (or other piercing or connecting element such as a cannula) secured to a distal end of the barrel 12; a stopper 13 slidably positioned within the barrel 12; and a plunger rod 14 engageable with the stopper. A removable needle cap 15 can be provided on the needle 11. The barrel 12 defines an inner fluid chamber. Forward movement (along said longitudinal axis) of the plunger rod 14 moves the stopper 13 from the proximal end 121 towards the distal end 122 (compare figure 1 and 2) which expels fluid from the barrel 12 through the needle 11. Rearward movement in the proximal direction of the plunger rod 14 will draw fluid into the barrel 12. Glass barrels are commonly used in prefillable or prefilled syringes, but plastic barrel are also known.

The syringe barrel 12 is coupled to a tubular body 2. The tubular body 2 is preferably an elongate, generally cylindrical piece, in particular made of plastic (preferably a semi-rigid plastic material such as polypropylene), which defines a generally cylindrical enclosure which advantageously shares the same longitudinal axis with the barrel 12. The tubular body 2 has proximal and distal open ends which provide access to the enclosure. A finger flange 20 extends radially outwardly from the tubular body 2 near the proximal open end thereof. The finger flange 20 and tubular body 2 are designed for easy handling as an injection is made so that an injection can be carried out using a single hand. The syringe assembly 10 also comprises a safety shield 3 for the needle 11, longitudinally movable with respect to the tubular body 2 - and consequently movable with respect to the barrel 12 - between a retracted position and an activated position, and a spring 4 configured for moving the shield 3. Preferably, the shield 3 is movable according to the same longitudinal axis as that of the barrel 12 and/or the tubular body 2. Like the tubular body 2, the shield 3 also has preferably an elongate, generally cylindrical piece, in particular made of plastic such as for example a semi-rigid plastic material such as polypropylene. In some embodiment, the spring 4 can be a metal coil spring, but other energy releasing element can be contemplated, such as deformable blades. In some embodiments, the length of the shield 3 is at least the length of the needle 11.

The syringe barrel 12 extends at least partially, and preferably substantially entirely, within the enclosure of the tubular body 2. The barrel 12 can be slidably engaged within the tubular body 2, and thus the syringe barrel 12 may include a radially outwardly extending flange for coupling onto an existing pre-fillable or prefilled syringe to the tubular body 2. In other embodiments, the tubular body 2 can be permanently secured on the barrel 12.

In any case, the needle safety shield 3 and the spring 4 can be placed inside the tubular body 2. An annular clearance is preferably kept in the enclosure between the tubular body 2 and the barrel 12 for housing the shield 3 and the spring 4. The safety shield 3 and the spring 4 are thus preferably configured and sized in diameter to be positioned within the tubular body 2, and to fit over the barrel 12.

In some embodiments, the needle 11 is covered by the safety shield 3 in the activated position and the needle 11 is un-covered by the safety shield 3 in the retracted position. By "covering" the needle 11 is meant enclosing the needle tip such that it is not exposed, preventing any risk of injury.

In other words, in the retracted position the shield 3 is partially, and preferably entirely, engaged within the enclosure of the tubular body 2, such that the needle 11 is exposed for use, while in the activated position (or "extended" position) the shield 3 axially protrudes from the tubular body 2 toward the distal end, hence sheathing the needle 11.

"First state" refers to the state of the present syringe assembly wherein the shield 3 is in the retracted state (see figure 1), and "second state" refers to the state of the present syringe assembly wherein the shield 3 is in the activated state (see figure 2).

The spring 4 urges said safety shield 3 towards said activated position. To this end, the tubular body 2 can include an end shoulder (close to its proximal open end) and in the retracted position, the spring 4 is compressed between the shield 3 and said end shoulder. Therefore, the spring 4 pushes the shield 3 in the longitudinal direction: "Relaxation" of the spring 4 causes a longitudinal movement of its distal extremity and then a longitudinal movement of the shield 3 so as to proximally extend from the distal end of the tubular body 2 and then reach the activated position. In other embodiments not shown, the proximal extremity of the spring 4 may directly be secured to the tubular body 2.

In some embodiments, the syringe assembly 10 includes a first locking mechanism 31 for locking the safety shield 3 in the retracted position. Indeed, without said first locking mechanism 31, the shield 3 could not stay in the retracted position, because of the spring 4 pushing action. In other words, said first locking mechanism 31 holds in place the shield 3 and/or the spring 4, and thus prevents the spring 4 from longitudinally moving the shield 3 towards the activated position. The force of the spring 4 is insufficient by itself to cause disengagement of the shield 3.

However, the first locking mechanism 31 can be manually releasable by a user so as to let the safety shield 3 reaches the activated position. In other words, the user can, by an action (see below) triggers the deactivation of the first locking mechanism 31 such that it stops restricting movement of the shield 3 and/or the spring 4. Thanks to the spring 4, the shield 3 switches from the retracted position to the activated position. We refer to as "activation of the shield" the manual release of the first locking mechanism 31, hence the word "activated" state of the shield 3 when extended.

The shield 3 is naturally held in the activated position by the spring 4, but a sufficient force could move the safety shield 3 back to the retracted position and then making apparent the needle 11. In some embodiments, the present syringe assembly can comprise a second locking mechanism 32 for locking the safety shield 3 in the activated position after the first locking mechanism 31 is released. A difference with the first locking mechanism 31 is that the second locking mechanism 32 is not manually releasable, even unintentionally by a user: the needle 11 cannot become exposed again, hence user and patient safety can be guaranteed.

To sum up, the safety shield 3 is initially held by the first locking mechanism 31 in the retracted position, then, after medication delivery completion, the user releases it and the safety shield 3 longitudinally moves up to engage the second locking mechanism 32, which prevents it from switching back to the restricted position.

The present assembly 10 includes the tubular body 2, the shield 3 and the locking mechanisms 31, 32, that is completely independent from the injection movement. For instance, while figure 2 represents the plunger rod 14 completely pushed down, i.e. the medication fully delivered, it is in practice possible to deploy the shield 3 regardless of the position of the plunger rod 14.

In particular, as depicted by figures 1 and 2:
- the safety shield 3 is able to rotate within the body 2 (which is typically the case if the shield 3 is also tubular), and has a distal part 302 axially protruding (in some embodiments a protrusion of at least 1 cm) from a distal part 202 of the body 2 for gripping the safety shield 3. More precisely, while most of the shield 3 is inside the body 2, and cannot be reached by the user, this distal part 302 extends out of the body 2, and therefore it can be grasped by the user, in particular for manually rotating the shield 3. Preferably, said distal part 302 of the safety shield 3 presents texture features (such as drops, as visible on figires 1 and 2) for increasing grip (i.e. adherence), and therfore easing grab and rotation by the user.
- the first locking mechanism 31 being configured to release the safety shield 3 when the safety shield 3 is rotated from a first angular position to a second angular position. It is to be understood that said first locking mechanism 31 does not prevent rotation of the shield 3, only axial translation.

Said first angular position can be arbitrary defined, it is only important that there exist at least one angular position (referred to as the first angular position) at which the safety shield 3 is locked in the retracted position, and at least one different position (referred to as the second angular position), different from the first angular position at which the safety shield 3 is released.

The first angular position is a "locked" angular position, and the second angular position is an "unlocked" angular position.

A rotation of the shield 3 (thanks to the distal part 302) allows moving from the first angular position to the second angular position, hence the wording "rotational trigger". The relative difference between the first angular position and the second angular position is for example 90°, but any angular value could be used. In some embodiments, there could be a plurality of first and/or second angular positions around the shield 3, for example:
- a first angular position at 0°;
- a second angular position at 90°;
- another first angular position at 180°;
- another second angular position at 270°.

The combination of the rotatable shield 3 and the body 2 allows simple yet very reliable locking mechanisms.

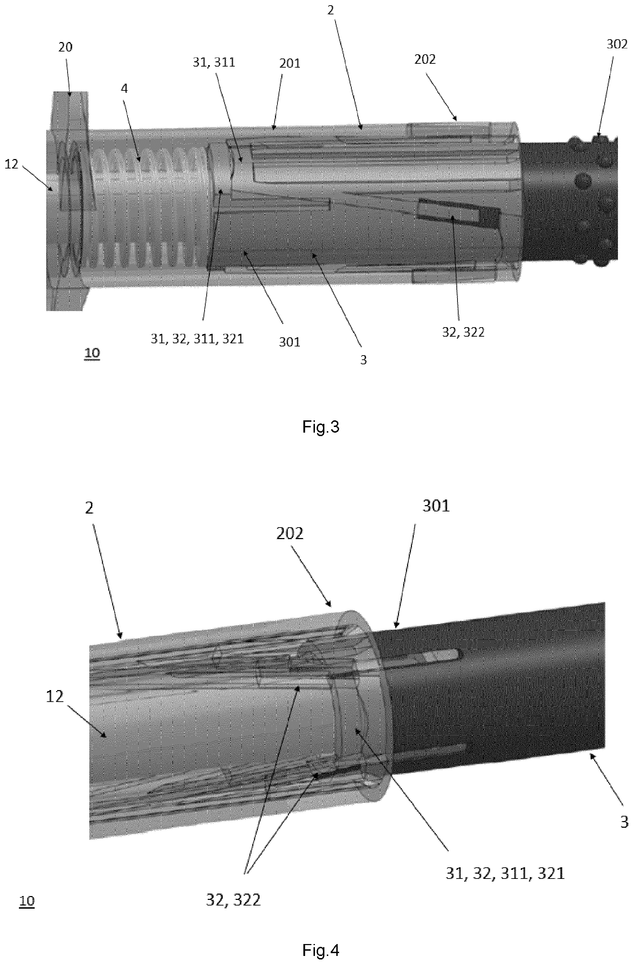

In some embodiements, it comprises complementary mating surfaces 311, 312 on the safety shield 3 and the body 2, see figure 3. More precisely, an outer surface of the shield 3 (typically at a proximal part 301 of shield 3) includes a first mating surface 311 (such as a tab) and an inner surface of the body 2 (typically at a proximal part 201 of the body 2) includes a complementary second mating surface 312 (such as another tab). The first and second mating surfaces 311, 312 are facing each other (and abutting) when the shield 3 is at the first angular position, which prevents the shield 3 from translating toward the activated position. Each mating surface 311, 312 has only a limited azimutal span, meaning that after a sufficient rotation of the shield 3 (up to the second angular position), they are no longer facing each other, and the release of the shield 3 is triggered. In some embodiments, there can be several first mating surfaces 311 and several mating surfaces 312, for example two pairs, diametrically opposed, see figures 3 and 4.

In another embodiements, the shield 3 may include a longitudinal slot, and the body 3 may include a radial pin engaging the longitudinal slot (protruding from said inner surface of the body 2, towards the center of the syringe assembly 1), and the first locking mechanism 31 is then configured to hold the radial pin at a proximal part of the longitudinal slot when the shield 3 is at the first angular position (for example a bayonet mount with a notch).

Similarly, there is a second locking mechanism 32, which is configured to receive and then hold a proximal part 301 of the safety shield 3.

In some embodiments, the second locking mechanism 32 can be a semi-flexible fastener, preferably with first retaining features 321 on the outer face of said proximal part 301 of the safety shield 3 which are forcedly deformed when the safety shield 3 moves from the retracted position towards the activated position. Said first retaining features 321 could also comprise the first mating surface 311, i.e. for instance a tab, see figure 4. In other words, the first mating surface is in this case part of both the first and the second locking mechanism 31, 32. This allows an overall simple and reliable architecture.

In the depicted embodiment, the proximal part 301 of the shield is divided (by longitudinal cuts) into a plurality of flexibe "strips" at the extremity of which the first mating surface(s) 311 is(are) located. The combination of the strips and the first mating surfaces 311 forms the first retaining features 321. Thus, when the safety shield 3 moves from the retracted position towards the activated position, each strip presenting a first mating surface 311 is forcedly deflected.

In some embodiments, the second locking mechanism 32 can comprise second retaining features 322 on the inner face of the distal part 202 of the body 2, which are configured to interact with said first retaining features 321 when the safety shield 3 moves from the retracted position towards the activated position for permanently and non-reversingly locking the safety shield 3 in the activated position after the first locking mechanism 31 is released. By "interaction", or "cooperation" it is meant that the first and second retaining features 321, 322 are also complementary and they can interlock when they enter into contact. In other words, during movement of the shield 3 the first retaining features 321 are forcibly deflected up to securely engage the second retaining features 322, and permanent lock is achieved because they are configured such that disengagement is impossible.

In the embodiment of figures 3 and 4, the second retaining features 322 are protrusions, for instance "teeth", extending from said inner surface of the distal part 202 of the body 2, and possibly V-shaped (i.e. not extending longitudinally but with a small angle). Under the force of the spring 4, the stripes of the first retaining features 321 are deflected when the first mating surface 311 (a tab) passes these teeth, and then the strips take back their position. At this stage the first mating surface 311 abuts the teeth of the second retaining features 322 (this is the "interaction" of the the second retaining features 322 with said first retaining features 321), and the strip cannot be forcedly deformed again by an axial force in the opposite direction.

The first locking mechanism 31 is manually releasable by the user (by rotating the shield 3), and/or the second locking mechanism 32 is not manually releasable by a user. Indeed, in the activated position, either the rotation of the shield 3 is inoperative as it does not have any impact on the second locking mechanism 32, or the rotation of the shield 3 becomes impossible; this is the case in the embodiment of figure 4, wherein the teeth of the second retaining features 322 extend on both the top and the sides of the first mating surface 311 of the first retaining features 321, thus preventing any movement of the shield 3 when in the activated position.

The strips could still theoretically be manually deflected by pressing them so as to pass again the teeth. However, the shield 3 is inside the body 2, and even in the activated state its proximal part 301 stays hidden. Therefore, the above-mentioned manipulations by a user are virtually impossible: the user cannot reach the first and second retaining features 321, 322, hence the second locking mechanism 32 is not manually releasable by a user and once locked is largely non-reversible.

## Claims

1. A syringe assembly for use in medication delivery comprising:
- a syringe barrel (12) having a needle (11) at a distal end (122) of the barrel (12);
- a tubular body (2) defining an enclosure, said barrel (12) extending at least partially within said enclosure;
- a safety shield (3) for the needle (11), longitudinally movable with respect to the tubular body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position,
- wherein the safety shield (3) is able to rotate within the body (2), and has a distal part (302) axially protruding from a distal part (202) of the body (2) for gripping the safety shield (3);
- the first locking mechanism (31) being configured to release the safety shield (3) when the safety shield (3) is rotated from a first angular position to a second angular position.

2. The syringe assembly of claim 1, comprising a second locking mechanism (32) for permanently and non-reversingly locking the safety shield (3) in the activated position after the first locking mechanism (31) is released.

3. The syringe assembly of claim 2, wherein the second locking mechanism (32) is configured to receive and then hold a proximal part (301) of the safety shield (3).

4. The syringe assembly of any one of claims 2 to 3, wherein the second locking mechanism (32) comprises first retaining features (321) on an outer face of said proximal part (301) of the safety shield (3) which are forcedly deformed when the safety shield (3) moves from the retracted position towards the activated position.

5. The syringe assembly of claim 4, wherein the second locking mechanism (32) further comprises second retaining features (322) on an inner face of the distal part (202) of the body (2), which are configured to interact with said first retaining features (321) when the safety shield (3) moves from the retracted position towards the activated position.

6. The syringe assembly of any one of claims 1 to 5, wherein the distal part (302) of the safety shield (3) presents texture features for increasing grip.

7. The syringe assembly of any one of claim 1 to 6, wherein the needle (11) is covered by the safety shield (3) in the activated position and the needle (11) is not covered by the safety shield (3) in the retracted position.

8. The syringe assembly of any one of claims 1 to 9, wherein the first locking mechanism (31) comprises complementary first and second mating surfaces (311, 312) respectively on the safety shield (3) and on the body (2).

9. The syringe assembly of claims 4 and 8 in combination, wherein the first mating surface (311) is also part of the first retaining features (321).

10. The syringe assembly of any one of claims 1 to 9, comprising a prefilled syringe (1) comprising said barrel (12), said needle (11), a stopper and a plunger rod (14).

11. A shielding system for a syringe (1) for use in medication delivery comprising a barrel (12) and a needle (11) at a distal end (122) of the barrel (12), said shielding system comprising:
- a tubular body (2) defining an enclosure, said barrel (12) extending at least partially within said enclosure;
- a safety shield (3) for the needle (11), longitudinally movable with respect to the tubular body (2) between a retracted position and an activated position;
- a spring (4) urging said safety shield (3) towards said activated position;
- a first locking mechanism (31) for locking the safety shield (3) in the retracted position, wherein the first locking mechanism (31) is manually releasable by a user so as to let the safety shield (3) reach the activated position;
- wherein the safety shield (3) is able to rotate within the body (2), and has a distal part (302) axially protruding from a distal part (202) of the body (2) for gripping the safety shield (3);
- the first locking mechanism (31) being configured to release the safety shield (3) when the safety shield (3) is rotated from a first angular position to a second angular position.

12. A method for operating a syringe assembly of any one of claims 1 to 10 or a shielding system of claim 11, comprising a step of rotating the safety shield (3) from the first angular position to the second angular position to trigger release of the first locking mechanism (31) so that the spring (4) moves the safety shield (3) from the retracted position towards the activated position.
